# EUROPEAN PATENT APPLICATION

(11) **EP 1 844 754 A2**
(43) Date of publication of application: **17.10.2007**
(21) Application number: 07005272.5
(22) Date of filing: 14.03.2007
(51) Int. Cl.: A61K 8/36, A61Q 19/00

(54) **Solubilization of acid ingredients**

(30) Priority: 13.04.2006 US 404148
(71) Applicant: L'Oréal, 75008 Paris (FR)
(72) Inventor: Fares, Hani, Somerset, NJ 08873 (US); Hansenne, Isabelle, Westfield, NJ 07090 (US); Guerrero, Rita Marie, Morris Plains, NJ 07950 (US); Russell, Michael, East Rutherford, NJ 07073 (US)
(74) Representative: Le Coupanec, Pascale A.M.P.

(57) **Abstract**

A topical composition containing from 0.5% to 15% by weight of at least one acid having a solubility, in water, at room temperature, of less than 0.002 g/ml, and from 10% to 99% by weight of at least one organic compound, liquid at room temperature, having at least one fatty group with a carbon chain length of from 12 to 24 carbon atoms, all weights being based on the weight of the composition.

## Description

A wide variety of acids is currently used for treating various skin conditions. Cosmetic and/or dermatological compositions containing acids are known in the art. They help to increase skin cell turnover and ultimately provide younger, fresher, healthier looking skin. Salicylic acid, its derivatives, and dioic acids are acids typically used as keratolytic agents which may be used for the treatment of acne, wrinkles, skin atrophy, psoriasis, hyperpigmentation. These acids are of great importance on account of their biological effects on the skin. However, formulating compositions containing these acids creates some issues, since they occur in crystalline form and are poorly soluble in water or in the oils traditionally used in the cosmetics field, such as mineral oils, petrolatum, and paraffin.

Attempts have been made to improve the solubility of acids in an aqueous phase. One way of improving their solubility involves the use of short-chain alcohol solvents such as ethanol or isopropanol. However, such short-chain alcoholic compositions can be harsh on the skin and can lead to irritation.

Accordingly, despite being able to solubilize acids of limited solubility in the aqueous phase of cosmetic compositions using methods such as described above, there is still a need for cosmetic compositions having improved anti-acne, anti-inflammatory, and/or anti-aging activity without the attendant harshness and skin irritation brought about by harsh solvents or solubilizers.

The present invention is directed to a topical composition comprising:
(a) from 0.5% to 15% by weight of at least one acid having a solubility, in water, at room temperature, of less than 0.002 g/ml; and
(b) from 10% to 99% by weight of at least one organic compound, liquid at room temperature, having at least one fatty group with a carbon chain length of from 12 to 24 carbon atoms, all weights being based on the weight of the composition.

The present invention is also directed to a process for solubilizing an acid in an organic compound involving the steps of:
(a) providing from 0.5% to 15% by weight of at least one acid having, a solubility, in water, at room temperature, of less than 0.002 g/ml;
(b) providing from 10% to 99% by weight of at least one organic compound, liquid at room temperature, having at least one fatty group with a carbon chain length of from 12 to 24 carbon atoms;
(c) combining (a) and (b) to form a mixture;
(d) heating the mixture to a temperature of from 60 to 65°C to form a heated mixture; and
(e) cooling the heated mixture to room temperature.

The present invention is also directed to a method, in particular a cosmetic method, for treating skin involving contacting the skin with a composition containing:
(a) from 0.5% to 15% by weight of at least one acid having a solubility, in water, at room temperature, of less than 0.002 g/ml; and
(b) from 10% to 99% by weight of at least one organic compound, liquid at room temperature, having at least one fatty group with a carbon chain length of from 12 to 24 carbon atoms, all weights being based on the weight of the composition.
   Lastly, the present invention is directed to the use of:

(a) from about 0.5% to about 15% by weight of at least one acid having a solubility, in water, at room temperature, of less than about 0.002 g/ml; and
(b) from about 10% to about 99% by weight of at least one organic compound, liquid at room temperature, having at least one fatty group with a carbon chain length of from about 12 to about 24 carbon atoms, all weights being based on the weight of the composition,
   for preparing a composition intended to treat acne, skin ageing or hyperpigmentation of the skin.

Applicants have thus unexpectedly discovered that organic compounds, liquid at room temperature, having at least one fatty group with a carbon chain length of from 12 to 24 carbon atoms, solubilize acids having a solubility, in water, at room temperature, of less than 0.002 mg/ml, which allow for the formulation of cosmetic compositions having improved anti-acne, anti-inflammatory, anti-aging and/or depigmenting activity without the attendant harshness typically associated with the use of such acids.

Other than in the operating examples, or where otherwise indicated, all numbers expressing quantities of ingredients and/or reaction conditions are to be understood as being modified in all instances by the term "about".

The composition of the present invention contains at least one acid having a solubility, in water, at room temperature, of less than 0.002 g/ml, which is solubilized in from 10% to 99% by weight of at least one organic compound, liquid at room temperature, having at least one fatty group with a carbon chain length of from 12 to 24 carbon atoms.

Acids having a solubility of less than 0.002g/ml

Examples of suitable acids include, but are not limited to, salicylic acid, unsaturated dioic acids, and their derivatives. Suitable acids are in particular chosen from salicylic acid, salicylic acid derivatives, unsaturated C₈-C₂₂ dioic acid, unsaturated C₈-C₂₂ dioic acid derivatives, and mixture thereof.

Salicylic acid

Salicylic acid or 2-hydroxybenzoic acid is a colorless, crystalline organic carboxylic acid that melts at 159°C. It has the following structure:

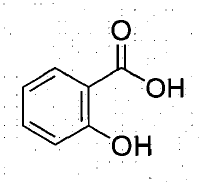

It is quite soluble in ethanol (1 g dissolves in 2.7 ml alcohol) and ether (1 g in 3 ml ether) but is only slightly soluble in water (1 g in 460 ml water). (The Merck Index, 12th edition, 1996).

Salicylic acid derivatives

Salicylic acid derivatives include, but are not limited to, those described in US Patent 4,767,750, and US Patent 5,558,871 the entire contents of which are hereby incorporated by reference. In particular, the salicylic acid derivative is preferred to have the formula:

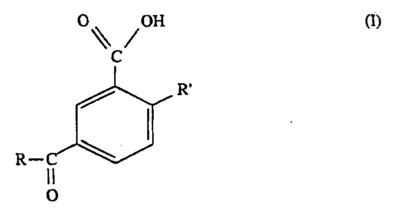

wherein:

R is a saturated linear, branched, or cyclic aliphatic group having from 3 to 11 carbon atoms; a linear branched, or cyclic unsaturated hydrocarbon group having from 3 to 17 carbon atoms and having one or more conjugated or unconjugated ethylenic double bonds; a saturated linear, branched, or cyclic aliphatic group having from 3 to 11 carbon atoms and substituted with at least one substituent selected from the group consisting of halogens, trifluoromethyl, hydroxyl, hydroxyl esterified with a carboxylic acid having from 1 to 6 carbon atoms, carboxyl, and carboxyl esterified with a lower alcohol having from 1 to 6 carbon atoms; or a linear, branched, or cyclic unsaturated hydrocarbon group having from 3 to 17 carbon atoms and having one or more conjugated or unconjugated ethylenic double bonds and substituted with at least one substituent selected from the group consisting of halogens, trifluoromethyl, hydroxyl, hydroxyl esterified with a carboxylic acid having from 1 to 6 carbon atoms, carboxyl, and carboxyl esterified with a lower alcohol having from 1 to 6 carbon atoms; and

R' is a hydroxyl group or an ester function of formula (II):

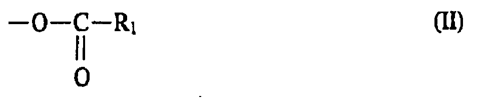

wherein R₁ is a saturated or unsaturated aliphatic group having from 1 to 18 carbon atoms.

These salicylic acid derivatives are less soluble in water than salicylic acid.

Preferred salicylic acid derivatives include 5-n-octanoylsalicylic acid and 5-n-dodecanoylsalicylic acid.

Unsaturated dioic acids and their derivatives

Examples of suitable unsaturated dioic acids and their derivatives include, but are not limited to, those described in US Patent 5,753,704, the entire content of which is hereby incorporated by reference. In particular derivatives include, but are not limited to, the derivatives comprising 15 to 22 carbon atoms in the main hydrocarbon chain. The term "main hydrocarbon chain", used with respect to dioic acid derivatives, is intended to refer to that part of the molecule situated between the oxygen atoms of the two carboxylic acid groups (or the derivatized remnants thereof). Thus, for example, derivatives having the formulae R-OOC-CH₂ - COO-R₁ and R-OOC-CH₂ -CH₂-COO-R₁ would be described as having C₃ and C₄ main hydrocarbon chains respectively.

The derivatives may be, for example, alcohols, substituted or unsubstituted amides, mono- or diesters (aryl or alkyl, especially lower alkyl esters) salts or mercapto derivatives.

In some embodiments, the unsaturated dioic acids employed in the compositions of the invention contain 8 to 22 carbon atoms, most preferably 16 or 18 carbon atoms. The unsaturated dioic acid derivative preferably contains 16 or 18 carbon atoms in the main hydrocarbon chain. A particularly preferred unsaturated dioic acid is 8-hexadecene 1,16 dicarboxylic acid known under the CTFA denomination of octadecenedioic acid. It has the following formula: 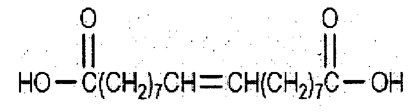 The acid may be a mixture of salicylic acid, 5-n-octadecenedioic acid and 8-hexadecene 1,16 dicarboxylic acid.

The amount of acids having a solubility of less than 0.002g/ml which can be dissolved depends on the organic compound liquid at room temperature used and the amount of the organic compound liquid at room temperature in the composition. The acids may be present in an amount of more than 0.01%, such as more than 0.5%, such as more than 1%, such as more than 5%, more than 10%, more than 15%, more than 20% based on the total weight of the composition containing the acid which is solubilized by an organic compound liquid at room temperature.

Organic compounds, liquid at room temperature

Organic compounds which are suitable for use in the present invention include, but are not limited to, fatty acids liquid at room temperature, fatty alcohols liquid at room temperature and fatty acid esters liquid at room temperature. In particular, the organic compound, liquid at room temperature, is chosen from fatty acids; fatty alcohols; esters of saturated and unsaturated, straight or branched, C₁₂ to C₂₀ fatty acids; and mixtures thereof.

Examples of suitable liquid fatty acids include, but are not limited to, C₁₂ to C₂₄ saturated or unsaturated, straight or branched fatty acids such as oleic acid, isostearic acid, linoleic acid, linolenic acid, ricinoleic acid and mixtures thereof, and in particular isostearic acid, oleic acid, linoleic acid, linolenic acid, and mixtures thereof.

Examples of suitable fatty alcohols, liquid at room temperature, include but are not limited to, those having from 12 to 24 carbon atoms, preferably from 12 to 22 carbon atoms, and more preferably from 16 to 22 carbon atoms. These liquid fatty alcohols may be straight or branched chain alcohols and may be saturated or unsaturated alcohols, preferably unsaturated alcohols. Liquid fatty alcohols useful in the invention may include, but are not limited to, oleyl alcohol, palmitoleyl alcohol, isostearyl alcohol, isocetyl alcohol, hexadecyl alcohol, octyldodecanol, linoleyl alcohol, linolenyl alcohol, lauryl alcohol, arachidyl alcohol, and mixtures thereof and in particular isostearyl alcohol, oleyl alcohol, hexadecyl alcohol, octyldodecanol, linoleyl alcohol, linolenyl alcohol, lauryl alcohol, arachidyl alcohol, and mixtures thereof.

Examples of suitable fatty acid esters, liquid at room temperature, include, but are not limited to, esters of fatty acids wherein said fatty acids have from 12 to 24 carbon atoms, are straight or branched, saturated or unsaturated. They include fatty acids having 12, 14, 16, 18 or 20 carbon atoms, examples of which may include oleic acid, isostearic acid, linoleic acid, linolenic acid, ricinoleic acid, arachidonic acid. The alcohol part of the ester of the fatty acid includes, but is not limited to, propylene glycol, glyceryl, isopropyl, isobutyl, and isopentyl. The esters of fatty acids, liquid at room temperature, include, but are not limited to, mono or di esters of propylene glycol such as propylene glycol monolaurate, propylene glycol ricinoleate, propylene glycol isostearate and mixtures thereof; mono, di or tri esters of glycerol such as glycerol isostearate, glyceryl diisostearate, glyceryl triisostearate and mixtures thereof; isopropyl isostearate; octyl dodecyl ricinoleate, isopropyl linoleate; isopropyl stearate and mixtures thereof.

Preferred organic compounds, liquid at room temperature, include oleic acid, isostearyl alcohol, glyceryl isostearate, propylene glycol isostearate, and mixtures thereof and in particular propylene glycol isostearate, glyceryl isostearate, and mixtures thereof.

In general, the organic compounds, liquid at room temperature, can be present in the composition in an amount of from 10 to 99% by weight, preferably from 10 to 60% by weight, more preferably from 10 to 50% by weight, and most preferably from 20 to 40% by weight, all weights being based on the total weight of the composition.

The compositions of the invention in particular the cosmetic compound and/or dermatological may be anhydrous in nature or comprise both oil and aqueous phases, in which case, the compositions may form emulsions/suspensions (e.g., oil-in-water, water-in-oil, and multiple emulsions) or be solutions or dispersions, and are formulated into products such as creams, lotions, gels or sticks.

The compositions of the present invention may further contain at least one suitable (e.g., cosmetically or dermatologically acceptable) ingredient, including additives and adjuvants, including, for example, polymers, waxes, thickeners, emulsifiers, moisturizers, colorants, dispersion enhancing agents (e.g., hydrolyzed corn starch), fillers (e.g., powders and mothers of pearl), sunscreen agents, preservatives, chelators (such as EDTA and salts thereof, particularly sodium and potassium salts), antioxidants (e.g., BHT, tocopherol), essential oils, fragrances, neutralizing or pH-adjusting agents (e.g., 2-amino-2-methyl-1,3-propanediol (AMPD) and sodium hydroxide), and cosmetically active agents and dermatological active agents, defoaming agents, emollients, vitamins, trace elements and essential fatty acids.

Examples of polymers, e.g., film forming polymers, include the following: proteins such as proteins of plant origin, such as wheat or soy proteins; gums (e.g., acacia gum); anionic, cationic, amphoteric or nonionic polymers of chitin or chitosan; plant-derived polymers such as hydrolyzed corn starch, cellulose polymers such as hydroxyethyl cellulose, hydroxypropyl cellulose, methyl cellulose, ethyl hydroxyethyl cellulose, carboxymethyl cellulose, and quaternized derivatives of cellulose; anionic polymers including acrylic and methacrylic polymers or copolymers such as polyacrylates or polymethacrylates, and salts (e.g., sodium salts) thereof; vinyl polymers, such as polyvinylpyrrolidones, copolymers of methyl vinyl ether and maleic anhydride, the copolymer of vinyl acetate and crotonic acid, copolymers of vinylpyrrolidone and vinyl acetate; copolymers of vinylpyrrolidone and caprolactam; and polyvinyl alcohols; and quaternized polymers (which are typically cationic polymers, but may also include amphoteric and zwitterionic polymers) such as polyquaternium-4, polyquaternium-6, polyquaternium-7, polyquaternium-8, polyquaternium-9, polyquaternium-10, polyquaternium-22, polyquaternium-32, polyquaternium-39, polyquaternium-44 and polyquaternium-47.

The compositions may contain a wax. Cosmetically acceptable waxes suitable for use in the invention are disclosed herein, and include natural and synthetic waxes alike. The wax may be added as a physical blend with one or more emulsifiers e.g., K82H (available from Koster Keunen).

Viscosity may be adjusted by adding a thickening/gelling agent capable of gelling an aqueous phase or a liquid fatty phase. Thickening/gelling agents may be chosen from thickening/gelling agents in polymeric form and thickening/gelling agents in mineral form. The thickening/gelling agent may perform its function via chemical reticulation and in some other cases, via physical reticulation.

Modified clays may be used as thickening/gelling agents, examples of which include hectorites modified with an ammonium chloride of a C₁₀ to C₂₂ fatty acid, such as hectorite modified with distearyldimethylammonium chloride, also known as quaternium-18 bentonite, such as the products sold or made under the names Bentone 34 by the company Rheox, Claytone XL, Claytone 34 and Claytone 40 sold or made by the company Southern Clay, the modified clays known under the name quaternium-18 benzalkonium bentonites and sold or made under the names Claytone HT, Claytone GR and Claytone PS by the company Southern Clay, the clays modified with stearyldimethylbenzoylammonium chloride, known as stearalkonium bentonites, such as the products sold or made under the names Claytone APA and Claytone AF by the company Southern Clay, and Baragel 24 sold or made by the company Rheox.

Other mineral thickening/gelling agents include silica, such as fumed silica. The fumed silica may have a particle size ranging from 5 nm to 200 nm.

Water-soluble thickening/gelling agents that may be used include polyvinylpyrrolidone (PVP); polyvinyl alcohol, crosslinked acrylates (e.g. Carbopol 982), hydrophobically-modified acrylates (e.g. Carbopol 1382); polyacrylamides such as, for example, the crosslinked copolymers sold under the names Sepigel 305 (CTFA name: polyacrylamide/C13-C14 isoparaffin/Laureth 7) or Simulgel 600 (CTFA name: acrylamide/sodium acryloyldimethyltaurate copolymer/isohexadecane/polysorbate 80) by SEPPIC; 2-acrylamido-2-methylpropanesulphonic acid polymers and copolymers, that are optionally crosslinked and/or neutralized; cellulose derivatives such as hydroxyethylcellulose, sodium carboxymethylcellulose, hydroxypropyl methylcellulose, hydroxypropyl cellulose, ethyl cellulose and hydroxymethyl cellulose; polysaccharides and gums, e.g., natural gums such as xanthan gum, sclerotium, carrageenan and pectin; polysaccharides such as starch and its derivatives; hyaluronic acid and its salts; clays, and, in particular, montmorillonites, hectorites, bentonites, and laponites; crosslinked polyacrylic acids, such as the "Carbopol" products from the company Goodrich, the polyglyceryl (meth)acrylate polymers sold under the names "Hispagel" or "Lubragel" by the companies Hispano Quimica or Guardian, crosslinked acrylamide polymers and copolymers, such as those sold under the names "PAS 5161" or "Bozepol C" by the company Hoechst, "Sepigel 305" by the company SEPPIC, crosslinked methacryloyloxyethyltrimethylammonium chloride homopolymers sold under the name "Salcare SC95" by the company Allied Colloid; and associative polymers and, in particular associative polyurethanes.

The thickening/gelling agent is generally present in an amount ranging from 0.05% to 20% by weight, and in some embodiments from 0.5% to 10% by weight, all weights being based on the total weight of the composition

Emulsifiers that may be used in the present invention include cosmetically acceptable non-ionic, anionic, cationic and amphoteric emulsifiers.

Compositions of the present invention may also contain a moisturizer. Examples include sodium lactate, mannitol, amino acids, hyaluronic acid, lanolin, urea, and mixtures thereof. Other examples include polyols such as glycerin, diglycerin, triglycerin, polyglycerin, polyethylene glycol, ethylene glycol, diethylene glycol, triethylene glycol, propylene glycol, dipropylene glycol, hexylene glycol, 1,3-butylene glycol, 1,4-butylene glycol and sorbitol.

These moisturizing agents are present in the compositions of the present invention in amounts generally ranging from 1.0% to 15%, and in some cases, from 2.0% to 10% by weight, all weights being based on the total weight of the composition.

Colorants may be chosen from the lipophilic dyes, hydrophilic dyes, traditional pigments, and nacres usually used in cosmetic or dermatological compositions, and mixtures thereof. The coloring agent may have any shape, such as, for example, spheroidal, oval, platelet, irregular, and mixtures thereof. Pigments may optionally be surface-treated e.g., with silicones, perfluorinated compounds, lecithin, and amino acids.

The liposoluble dyes include, for example, Sudan Red, D&C Red 17, D&C Green 6, soybean oil, Sudan Brown, D&C Yellow 11, D&C Violet 2, D&C Orange 5, quinoline yellow and annatto. The water-soluble dyes are, for example, beetroot juice or methylene blue.

Suitable pigments may be chosen from white pigments, colored pigments, inorganic pigments, organic pigments, coated pigments, uncoated pigments, pigments having a micron size and pigments not having a micron size. Suitable inorganic pigments may include titanium dioxide, optionally surface-treated, zirconium oxide, zinc oxide, cerium oxide, chromium oxide, manganese violet, ultramarine blue, chromium hydrate, and ferric blue. Suitable organic pigments may include carbon black, pigments of D&C type, lakes based on cochineal carmine, lakes based on barium, lakes based on strontium, lakes based on calcium, and lakes based on aluminum.

Suitable nacreous pigments may, for example, be chosen from white nacreous pigments such as mica coated with titanium and mica coated with bismuth oxychloride, colored nacreous pigments such as titanium mica with iron oxides, titanium mica with, for example, ferric blue and/or chromium oxide, titanium mica with an organic pigment of the type mentioned above, as well as nacreous pigments based on bismuth oxychloride, interferential pigments, and goniochromatic pigments.

In general, colorants may be present in an amount ranging from 0.01% to 50%, and in some embodiments from 0.01% to 30%, from 0.01% to 20%, and from 3% to 10%, by weight, all weights being based on the total weight of the composition.

The compositions of the present invention may also contain dispersion enhancing agents such as polysaccharide resins, e.g., KM 13, available from KAMA International Corp. (Duluth, GA).

Fillers, powders and mothers-of-pearl may also be added to the formulations, typically to modify the texture of the composition and the matteness/gloss effect. Fillers should be understood to mean lamellar or non-lamellar, inorganic or synthetic, colorless or white particles. Mothers-of-pearl should be understood to mean iridescent particles produced especially by certain mollusks in their shell or else synthesized. Representative examples of these ingredients include mica, silica, kaolin, iron oxides, titanium dioxide, polyamide powders, polyamide powders, for instance Nylon^{®} (Orgasol from Atochem), poly-alanine powders, polyethylene powders, tetrafluoroethylene polymer powders, for instance Teflon^{®}, starch (e.g., hydrolyzed corn starch), boron nitride, hollow polymer microspheres such as those of polyvinylidene chloride/acrylonitrile, for instance Expancel^{®} (Nobel Industrie), acrylic powders such as Polytrap^{®} (Dow Corning), polymethyl methacrylate particles and silicone resin microbeads (for example Tospearls^{®} from Toshiba), magnesium hydrocarbonate, hydroxyapatite, hollow silica microspheres (Silica Beads^{®} from Maprecos), and glass and ceramic microcapsules. Filler(s), if present, are in amounts generally ranging from 0.1% to 25%, and in some embodiments from 1% to 20% by weight, by weight, all weights being based on the total weight of the composition.

Representative examples of sunscreen agents may be chosen from organic and inorganic sunscreen agents. Organic sunscreen may be chosen in particular from anthranilates; cinnamic derivatives; dibenzoylmethane derivatives; salicylic derivatives; camphor derivatives; triazine derivatives; benzophenone derivatives; β,β-diphenylacrylate derivatives; benzotriazole derivatives; benzalmalonate derivatives; benzimidazole derivatives; imidazolines; bisbenzoazolyl derivatives; p-aminobenzoic acid (PABA) derivatives; methylenebis(hydroxyphenylbenzotriazole) derivatives; benzoxazole derivatives; screening polymers and screening silicones; dimers derived from α-alkylstyrene; 4,4-diarylbutadienes, and their mixtures.

Inorganic sunscreen agents may be selected from the group consisting of particles with a mean size generally between 5 nm and 100 nm, preferably between 10 nm and 50 nm. These particles are formed from coated or uncoated metal oxides, such as, for example, titanium oxide (amorphous or crystalline in the rutile and/or anatase form), iron oxide, zinc oxide, zirconium oxide or cerium oxide, which are all UV sunscreens well known per se. Conventional coating agents are, furthermore, alumina and/or aluminum stearate. Sunscreen agents suitable for use in the composition of the present invention are described in US 6,916,464, the entire content of which is incorporated herein by reference.

Representative examples of preservatives include alkyl para-hydroxybenzoates, wherein the alkyl radical has from 1, 2, 3, 4, 5 or 6 carbon atoms and preferably from 1 to 4 carbon atoms e.g., methyl para-hydroxybenzoate (methylparaben), ethyl para-hydroxybenzoate (ethylparaben), propyl para-hydroxybenzoate (propylparaben), butyl para-hydroxybenzoate (butylparaben) and isobutyl para-hydroxybenzoate (isobutylparaben), and phenoxyethanol. Mixtures of preservatives are also useful, e.g., the mixture of methylparaben, ethylparaben, propylparaben and butylparaben sold under the name Nipastat by Nipa, the mixture of phenoxyethanol, methylparaben, ethylparaben, propylparaben and butylparaben, also sold by Nipa under the name Phenonip, and the mixture of phenoxyethanol, methylparaben, isopropylparaben, isobutylparaben and butylparaben, sold by ISP under the name Liquapar Optima. The preservative may be present in an amount generally ranging from 0.01% to 15% by weight, all weights being based on the total weight of the composition.

The compositions of the present invention may also contain antioxidants. Antioxidants are ingredients used in cosmetic or dermatological compositions to prevent or slow down product spoilage from rancidity. They may also be used for their activity as free radical scavengers. Typical antioxidants used generally include ascorbic acid and its derivatives, BHA, BHT, ferulic acid and its derivatives, tocopherol and its derivatives, as well as those described in the International Cosmetic ingredient Dictionary and Handbook, Ninth edition, Vol. 4, pages 2883 and 2884, which is incorporated herein by reference.

Moreover, the compositions of the present invention may contain, in the fatty phase, in addition to the acid having a solubility, in water, at room temperature, of less than 0.002 g/ml, and the organic compound liquid at room temperature, mineral oils (liquid petrolatum), synthetic oils, silicone oils (cyclomethicone or dimethicone), perfluorinated oils (perfluoro polyethers), fatty alcohols and fatty acids.

Of course, a person skilled in the art will take care to choose this or these possible additional compounds and/or their amounts so that the advantageous properties of the compositions according to the invention are not, or not substantially, detrimentally affected by the envisaged addition.

Such compositions can typically be used for treating the body and face, including the scalp and nails, and more especially for treating acne, skin ageing (wrinkles, fine lines, complexion) or hyperpigmentation of the skin.

Other features of the invention will become apparent in the course of the following descriptions of exemplary embodiments which are given for illustration of the invention and are not intended to be limiting thereof.

EXAMPLES

The present invention is further described in terms of the following non-limiting examples. Unless otherwise indicated, all parts and percentages are on a weight-by-weight percentage basis.

EXAMPLE 1

In this example, the solubility of salicylic acid in different liquid materials was compared with mineral oil.

Preparation of highly concentrated salicylic acid solutions

Salicylic acid was mixed with various organic compounds, liquid at room temperature, and heated to 60 °C. The solution was stirred until all the salicylic acid was dissolved and then cooled to room temperature with stirring. The solution was left undisturbed for 2 hours and observed for crystallization. The following solubilities were obtained:
Oleic acid: 5.6% w/w
Isostearic acid: 6.5% w/w
Glyceryl isostearate: 8.25% w/w
Propylene glycol isostearate: 9.9% w/w
Isostearyl alcohol: 9.9% w/w
C₁₂-C₁₅ alkyl benzoate: 4.8% w/w

The results are expressed by weight of the acid in the dissolving organic compound liquid at room temperature. The results from the above experiments show much improved solubility when using organic compounds, liquid at room temperature, according to the invention. The inventive compositions show solubilities in excess of 4%, such as 4.8% or greater.

EXAMPLE 2

| Phase | Ingredient | %w/w |
|---|---|---|
| A1 | Water | 67.10 |
| | Cucumber Extract | 0.10 |
| | Alcohol | 5.00 |
| | Preservative | 0.50 |
| A2 | Thickener | 0.70 |
| B | Propylene glycol isostearate | 20.00 |
| | Preservative | 0.50 |
| | Salicylic Acid | 1.50 |
| | 5-n-octanoylsalicylic | 0.50 |
| | Octadecenedioic Acid | 2.00 |
| C | Potassium hydroxide | 0.60 |
| D | Polysorbate 80 | 0.50 |
| E | Thickener | 1.00 |
| | TOTAL | 100.00 |

This composition was made according to the following protocol:

Phase A1: water was added to main vessel. Slow mixing was started with the homogenizer. The preservative, alcohol and cucumber extract were added and mixed well. Heating to 70-75 °C was started.

Phase A2: The thickener was added into the main vessel, and mixed until it was well hydrated.

Phase B: All ingredients in phase B were added into another vessel and mixed well with magnetic stir bar. The mixture was heated to a temperature of 60-65 °C. The mixing was maintained until all ingredients were melted and that the phase was uniform.

Phase B was added to Phase A1+A2 into the main vessel and mixed well for 10 minutes. The mixture of Phase A1+A2 and Phase B was cooled to a temperature of 50-55 °C.

Phase C was added to the main vessel and mixed well. The batch was maintained at a temperature of 50-55 °C.

Phase D was added to main vessel and mixed well.

Phase E was sprinkled into the main vessel and mixed well until fully hydrated.

The mixture was cooled to 25 °C.

The composition obtained is a skin care gel for acne treatment or for wrinkle treatment.

EXAMPLE 3

| Phase | Ingredient | %w/w |
|---|---|---|
| A1 | Water | 67.10 |
| | Preservative | 0.50 |
| A2 | Thickener | 0.70 |
| B | Propylene glycol isostearate | 20.00 |
| | Preservative | 0.50 |
| | Salicylic Acid | 1.50 |
| | 5-n-Octanoylsalicylic | 0.50 |
| | Octadecenedioic Acid | 2.00 |
| | Polysorbate 80 | 0.50 |
| C | Potassium hydroxide | 0.60 |
| D | Thickener | 1.00 |
| E | Cucumber extract | 0.10 |
| | Alcohol | 5.00 |
| | TOTAL | 100.00 |

This composition was made according to the following protocol:

Phase A1: water was added to the main vessel. Slow mixing with homogenizer was started and the preservative was added and mixed well. The mixture was heated to a temperature of 70-75 °C.

Phase A2: The thickener was sprinkled into the main vessel and mixed until the thickener was well hydrated.

Phase B: All the ingredients in phase B were added to a beaker, mixed with a magnetic stir bar, and heated to a temperature of 60-65 °C. Mixing was continued until all ingredients were melted and that the phase was uniform.

Phase B was added to Phase A1+A2 into the main vessel and mixed well for 10 minutes. The mixture Phase A1 + A2 and Phase B was then cooled to a temperature of 50-55 °C.

Phase C was added to the main vessel and mixed well. The batch was cooled to a temperature of 50-55 °C.

Phase D was sprinkled into the main vessel and mixed well ensuring that the thickener is fully hydrated. The batch was cooled to a temperature of 25 °C.

Phase E was added to the main vessel and mixed well.

The composition obtained is a skin care gel for acne treatment.

## Claims

1. A topical composition comprising:
(a) from about 0.5% to about 15% by weight of at least one acid having a solubility, in water, at room temperature, of less than about 0.002 g/ml; and
(b) from about 10% to about 99% by weight of at least one organic compound, liquid at room temperature, having at least one fatty group with a carbon chain length of from about 12 to about 24 carbon atoms, all weights being based on the weight of the composition.

2. The topical composition according to Claim 1 wherein the acid is chosen from salicylic acid, salicylic acid derivatives, unsaturated C₈-C₂₂ dioic acid, unsaturated C₈-C₂₂ dioic acid derivatives, and mixtures thereof.

3. The topical composition according to Claim 1 or 2 wherein the acid is salicylic acid.

4. The topical composition according to any one of Claims 1 to 3 wherein the acid is 5-n-octanoylsalicylic acid.

5. The topical composition according to any one of Claims 1 to 4 wherein the acid is 8-hexadecene 1,16 dicarboxylic acid.

6. The topical composition according to any one of Claims 1 to 5 wherein the acid is a mixture of salicylic acid, 5-n-octanoylsalicylic acid and 8-hexadecene 1,16 dicarboxylic acid.

7. The topical composition according to any one of Claims 1 to 6, wherein the organic compound, liquid at room temperature, is chosen from fatty acids; fatty alcohols; esters of saturated, unsaturated, straight or branched C₁₂ to C₂₀ fatty acids and mixtures thereof.

8. The topical composition according to any one of Claims 1 to 7, wherein the organic compound, liquid at room temperature, is chosen from isostearic acid, oleic acid, linoleic acid, linolenic acid and mixtures thereof.

9. The topical composition according to any one of Claims 1 to 8, wherein the organic compound, liquid at room temperature, is chosen from isostearyl alcohol, oleyl alcohol, hexadecyl alcohol, octyldodecanol, linoleyl alcohol, linolenyl alcohol, lauryl alcohol, arachidyl alcohol and mixtures thereof.

10. The topical composition according to any one of Claims 1 to 9, wherein the organic compound, liquid at room temperature, is chosen from propylene glycol, glyceryl, isopropyl, isobutyl and isopentyl esters of fatty acids, and mixtures thereof.

11. The topical composition according to any one of Claims 1 to 10, wherein the organic compound, liquid at room temperature, is chosen from propylene glycol isostearate, glyceryl isostearate, and mixtures thereof.

12. A process for solubilizing an acid in an organic compound comprising at least the steps of:
(a) providing from about 0.5% to about 15% by weight of at least one acid having a solubility, in water, at room temperature, of less than about 0.002 g/ml;
(b) providing from about 10% to about 99% by weight of at least one organic compound, liquid at room temperature, having at least one fatty group with a carbon chain length of from about 12 to about 24 carbon atoms;
(c) combining (a) and (b) to form a mixture;
(d) heating the mixture to a temperature of from about 60 to about 65°C to form a heated mixture; and
(e) cooling the heated mixture to room temperature.

13. The process accordingly to Claim 12, wherein the acid is as defined in anyone of Claims 2 to 6.

14. The process according to Claim 12 or 13, wherein the organic compound, liquid at room temperature, is as defined in any one of Claims 7 to 11.

15. A cosmetic method for treating skin comprising contacting the skin with a composition containing:
(a) from about 0.5% to about 15% by weight of at least one acid having a solubility, in water, at room temperature, of less than about 0.002 g/ml; and
(b) from about 10% to about 99% by weight of at least one organic compound, liquid at room temperature, having at least one fatty group with a carbon chain length of from about 12 to about 24 carbon atoms, all weights being based on the weight of the composition.

16. The cosmetic method according to Claim15, wherein the acid is as defined in any one of Claims 2 to 6.

17. The cosmetic method according to Claim15 or 16, wherein the organic compound, liquid at room temperature, is as defined in any one of Claims 7 to 11.

18. Use of:
(a) from about 0.5% to about 15% by weight of at least one acid having a solubility, in water, at room temperature, of less than about 0.002 g/ml; and
(b) from about 10% to about 99% by weight of at least one organic compound, liquid at room temperature, having at least one fatty group with a carbon chain length of from about 12 to about 24 carbon atoms, all weights being based on the weight of the composition,
for preparing a composition intended to treat acne, skin ageing or hyperpigmentation of the skin.
